# EUROPEAN PATENT SPECIFICATION

(11) **EP 1 847 212 B1**
(45) Date of publication and mention of the grant of the patent: **03.12.2008**
(21) Application number: 06008342.5
(22) Date of filing: 21.04.2006
(51) Int. Cl.: A61B 1/00, A61B 1/05, A61B 1/06

(54) **Endoscope with a digital view system**
Endoskop mit einem digitalen Betrachtungssystem
Endoscope avec une système numérique de vision

(43) Date of publication of application: 24.10.2007
(73) Proprietor: Fondazione Torino Wireless, 10129 Torino (IT)
(72) Inventor: Pasero, Eros, 10131 Torino (IT); Meindl, Tassilo, 10139 Torino (IT); Omede', Marco, 10135 Torino (IT); Moniaci, Domenico, 10100 Torino (IT)
(74) Representative: Grünecker, Kinkeldey, Stockmair & Schwanhäusser Anwaltssozietät

(56) References cited:
- EP-A- 0 592 194
- WO-A-20/04112594
- DE-A1- 4 102 196
- US-A- 4 641 635
- US-A1- 2002 188 177
- PATENT ABSTRACTS OF JAPAN vol. 1998, no. 09, 31 July 1998 (1998-07-31) -& JP 10 108828 A (OLYMPUS OPTICAL CO LTD), 28 April 1998 (1998-04-28)

## Description

### FIELD OF THE PRESENT INVENTION

The invention concerns an endoscope with a digital view system such as a digital camera, with a housing having a longitudinal axis, with a lens attached to the housing and with an electronic image sensor positioned in the interior of the housing for converting a picture delivered through the lens into a digital format.

### DESCRIPTION OF THE PRIOR ART

In the state of the art, endoscopes are known. Endoscopes with digital cameras are also known. Those endoscopes normally have a fixed focus, that means that the distance between a lens in the endoscope and the camera system in the endoscope does not change. On the other hand, endoscopes are known where the distance between an image sensor of the camera in the endoscope and the lens of the endoscope differs. The changing of distance can be conducted automatically or manually.

Due to limitations of common endoscopes, that means endoscopes without digital image technique, digital cameras in combination with endoscopes have found wide application in order to improve diagnostic methods.

Digital cameras for endoscopes comprising a base station such as a personal computer (PC) which provides power to a hand piece being part of the housing of the endoscope. The hand piece usually comprises an image sensor such as a charge coupled device (CCD) or a complimentary metal oxide semi-conductor (CMOS) camera, a light source, a video processing micro-processor and user-interfacing such as buttons for snapshot functionality. In order to provide a good image quality optical systems sometimes lenses and mirrors have to be provided which focus the image on the image sensor. Currently, there can be seen a shift from the classical CCD based system to CMOS based system, which simplifies the interfacing to modem personal computers via connection standards such as USB or wireless fidelity (WiFi).

Generally, three types of digital cameras for endoscopy exist: fixed focus, manual focus and autofocus cameras. Due to the consumers preferences nowadays, simple manual mechanisms or autofocus cameras are a must. However, compared to commercially available digital cameras, focus mechanisms for endoscopy cameras are limited by their small size in the head part of the hand piece, which is extremely critical in dental, rectal and/or vaginal endoscopy. Endoscopes with a fixed focus are pre-configured for certain optical distances, such as for inter-oral cameras for macro teeth pictures. However, this extremely limits the applicability of said type of cameras for other utilizations, such as complete panorama views.

Manual focus endoscopes give the opportunity to adjust the lens distance. However, this solution is improvised and not comfortable. The improved mechanical system controls the distance of the lens to an image sensor, such as a circuit, by indirectly moving the lens by help of a small motor. However, an inter-oral camera space is limited and therefore those systems are not reliable or mechanically elaborate.

In the state of the art, autofocus endoscopes have cameras, which are fixed vertically, whereby the image is projected using a mirror and have a complicated optical system onto the camera. In the endoscopes, relevant for this invention, housings consist of a hand part, which is held in the operator's hand, and a head part, which is introduced into a human or animal body, especially in some orifice. In the head part of these endoscopes a lens is fixed. The lens is in the side wall of the head part. The image taken by the endoscope is of an area perpendicular to the longitudinal axis through the endoscope, especially through the head part. The positioning of a lens in the very tip of the head part is, however, also possible. The right focus can be achieved in this system by either moving the camera or lenses in the optical path. This avoids small sized mechanical systems in the head part, because of the camera, the focusing system can be mounted in a larger hand part, also known as a handle. Further on, autofocus functionality can be achieved in said solution by standard methods, such as direct reflection or indirect image processing algorithms. However, said solutions are quite complex and therefore expensive and cost intensive.

An endoscope disclosing the features of the preamble of claim 1 is known from document JP 10108828.

### SUMMARY OF THE PRESENT INVENTION

It is therefore an object of the current invention to overcome the drawbacks of the state of the art. In particular, it is an object of the present invention to provide an endoscope characterized by reduced complexity, low costs and improved reliability.

To this end, in the endoscope according to the present invention, instead of moving the lens, the mechanical system is used in order to move the circuit in which the camera is amounted.

In particular, according to the present invention there is provided an endoscope as claimed in claim 1, namely an endoscope with a digital view system, such as a digital camera, with a housing having a longitudinal axis, with a lens attached to the housing and with an electronic image sensor positioned in the interior of the housing for converting a picture delivered through the lens Into a digital format, wherein the image sensor is moveable transverse to the longitudinal axis and the image sensor is fixed to a carrier, whereby the carrier is pivotally attached to the housing

The invention overcomes the drawbacks of the state of the art by a simple and low cost mechanical mechanism. In fact, due to the fact that, instead of moving the lens, the mechanical system is used In order to move the circuit in which the camera is mounted, said movement can be controlled from the handle of the endoscope, where space limitations are not as stringent as in the head part. The current invention considerably lowers costs for an endoscope with good properties.

Preferable embodiments are claimed in the dependent claims. The preferable embodiments are also described in the following.

It is advantageous to have another embodiment in which the image sensor is moveable perpendicular to the longitudinal axis. Rectangular movements and the therefore necessary guides are easy to manufacture and very precise.

If the image sensor is fixed to a carrier being parallel to the longitudinal axis, the carrier can be moved to result the movement of the image sensor. The necessary geometrical interrelationships are therefore more positive.

To be able and deliberately focused on a special object, it is advantageous to provide a focus regulation unit in the housing, to change the distance between the lens and the image sensor.

If the image sensor comprises a circuit, common elements can be used to provide a digital view system, such as a digital camera in the endoscope.

If the focus regulation unit allows a skew of the carrier relative to the lens, the focus can be advantageously easily changed in this embodiment.

As a screw is easy to manufacture and inexpensive to provide, it is advantageous to configure another embodiment, in which the focus regulation unit comprises an adjustment element, such as a wheel or a screw, moving the carrier relevant to the housing.

If the endoscope comprises an interface for relaying the data of the image sensor circuit to a processing unit, such as a personal computer or a display, the picture received by the image sensor circuit through the lens in the interior of the body, for example, can be relayed to the interface and then to a personal computer or a display, so that the image can be easily viewed apart from the object which is probed.

To relay the data easily from one place to the other, it is advantageous to provide an interface, which is a USB connector and/or a wireless communication module.

If the housing comprises a head part and a hand part, whereby the head and hand parts are preferably positioned consecutively relative to the longitudinal axis, a long and easy to handle endoscope is resulting in this preferable embodiment.

To be able to enter the endoscope in small orifices, it is advantageous to provide a head part having a smaller diameter than the hand part, whereby the head part is suitable to be inserted in orifices of a human or animal body.

If the lens is fixed to the head part to allow pictures to be transmitted in the interior of the housing, an unwanted movement of the lens relative to the image sensor can be avoided, even if pressure is applied to the lens. The focus therefore stays the same.

If the focus regulation unit is positioned in the hand part, it is advantageous that more space can be used for the movement mechanics.

To achieve a simple usability of the endoscope, it is advantageous that the screw or the wheel is positioned on the interior of the hand part and is in contact with the carrier, for traversing the carrier relative to the housing of the hand part.

If a support member is positioned in the housing, separately to the carrier, a lot of advantageous embodiments can be achieved.

With the carrier being pivotally attached to the housing a very low cost version of the endoscope is producible.

To avoid distortions, it is advantageous, if the carrier is pivotally attached with an end, distant to the lens, to the interior of the hand part.

If the carrier is moveable parallel to the support member, distortions can be totally avoided in this advantageous embodiment.

To provide the relaying of the data, it is advantageous, that the interface is attached to the support member and/or an inter-linkage is linking the image sensor to the interface.

To get pictures even of dark objects, it is advantageous, that at least one lamp, preferably a LED or even two LED's, is attached together with the lens in one opening of the housing and preferably covered by a flush covering of the housing.

If the covering is of acrylic glass, pictures are still of good quality and injuries in the orifice can be avoided.

If the endoscope comprises a mode switch to chose between an autofocus and a manual focus mode the versatility of the endoscope is maximized.

### BRIEF DESCRIPTION OF THE DRAWINGS

In the following, a description will be given with reference to the drawings of particular and/or preferred embodiments of the present invention; it has, however, to be appreciated that the present invention is not limited to the embodiments disclosed, but that the embodiments disclosed only relate to particular examples of the present invention, the scope which is defined by the appended claims. In the drawings, the figures show:
FIG. 1 a state of the art endoscope with a fixed focus in a schematic sideview;
FIG. 2 a schematic side part view of the head part of the state of the art fixed focus endoscope of Fig. 1;
FIG. 3 a schematic part view of the head part and a small area of a hand part of an autofocus endoscope of the state of the art;
FIG. 4 a schematic side view of a first embodiment of the invention, to show the principal;
FIG. 5 a schematic side view of an endoscope according to the invention with a support member, carrying the image sensor and being pivotally attached to the housing of the endoscope;
FIG. 6 a schematic side view of another embodiment of the invention;
FIG. 7 a side view of another embodiment of the invention shown schematically;
FIG. 8 a schematic side view of the detail in VIII of Fig. 7;
FIG. 9 another embodiment of the invention in a schematic side view; and
FIG. 10 a diagram showing the image quality information.

### DETAILED DESCRIPTION

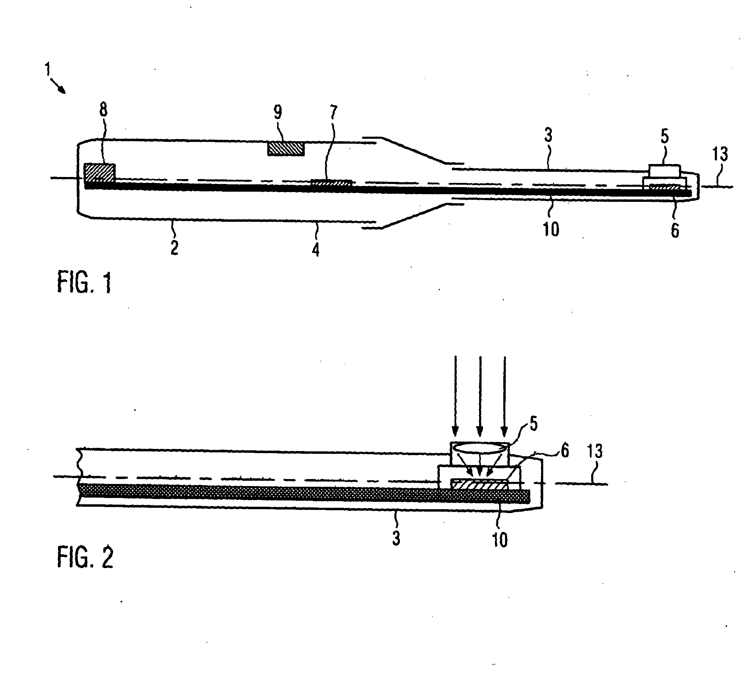

FIG. 1 shows an endoscope 1 with a fixed focus. The endoscope comprises a housing 2. The housing 2 comprises a head part 3 and a hand part 4. The hand part 4, also known as a handle, is the part of the housing 2 of the endoscope 1 which is held by an operator. The head part 3 is entered into an orifice of a human or an animal body. The material used for the housing is preferably of synthetic material. A lens 5 is included in the housing 2, namely in the side of the head part 3, near the tip. The lens 5 is fixed to the head part. Through the lens, light rays may reach the interior of the housing 2. The light rays reach in Fig. 1 directly an image sensor 6. The image sensor 6 is part of the digital view system, such as a digital camera. The light rays relay an image from the outside of the housing 2 and are transformed in electronical information, such as digital information, which is transferred to a video processing unit 7. The video processing unit 7 changes the data and transverses it to an interface 8. The interface may be a USB port or a wireless connection element, such as an element used for WiFi. In the side of the hand part 3, a button 9 is included, which is pressable and due to the pressure applied to the button 9, a picture is taken by the endoscope 1.

In the other figures, the same reference numbers are used for the same elements.

In Fig. 2 the tip area of the head part 3 of the endoscope 1 is shown. As can be seen in Fig. 2 and Fig. 1 the image sensor 6 is mounted on a carrier 10 in the interior of the housing 2. Light rays transfer the lens 5 and reach the image sensor 6. The state of the art endoscope has a fixed focus, meaning that the distance between the image sensor 6 and the lens cannot be changed. Therefore, an operator has to change the distance between the lens 5 and the object which is viewed.

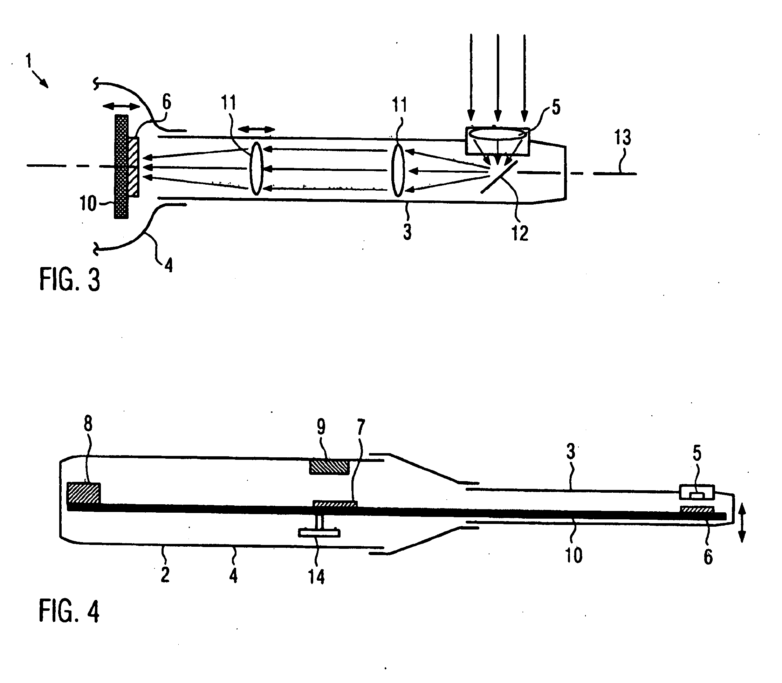

In Fig. 3 an autofocus endoscope 1, as known from the state of the art, is shown. Apart from the lens in the side of the head part 3, focus lenses 11 are in the interior of the head part. The light rays are turned around by use of a mirror 12. The light rays reach the image sensor 6 being fixed on the carrier 10. The carrier 10 and the thereon fixed image sensor are movable in a longitudinal direction. In the Figs. 1 - 3 the longitudinal axis has the reference number 13.

Fig. 4 shows the preferred embodiment of the endoscope 1, wherein a CMOS active pixel sensor chip having an analogue output is utilized. Analog to digital conversion in image processing functions may be implemented in the camera chip. Further, on a onboard video compression and processing chip, such as the video processing unit 7, may be provided in order to reduce data to be transmitted and improve image quality onboard.

This significantly reduces space compared to classical CCD based systems. This might be particularly interesting in case of wireless or low-speed interfacing to a base station.

Such a base station may be a personal computer (PC) or a display. Further, on the lower power consumption of CMOS image sensors 6 provides for a more efficient power management in case of power supply from a battery and wireless data transmission. The invention of this embodiment provides a dental camera in the endoscope 1, having a single lens 5. The image sensor 6 is configured as a circuit on a carrier 10. The circuit is mounted in a distance of the single lens 5, in order to focus the image on the camera for extra - oral (infinite distance) images. Positioning the carrier 10 and the circuit closer to the lens 5 provides for focus for inter - oral and macro images. In this simplest solution the user can perform said positioning of the circuit by turning a wheel of a focus regulation unit 14, which is connected to an internal screw, which is fixed towards the circuit and therefore regulates the distance between the camera and the lens 5.

This allows for a very low - cost adjustment of a focus of the inter - oral camera of the endoscope 1.

By moving the wheel of the focus regulation unit 14 the carrier 10 is moved and the thereon fixed image sensor, namely the circuit, is moved, as can be seen at the tip area of the head part 3, towards the lens 5 or from the lens 5 away.

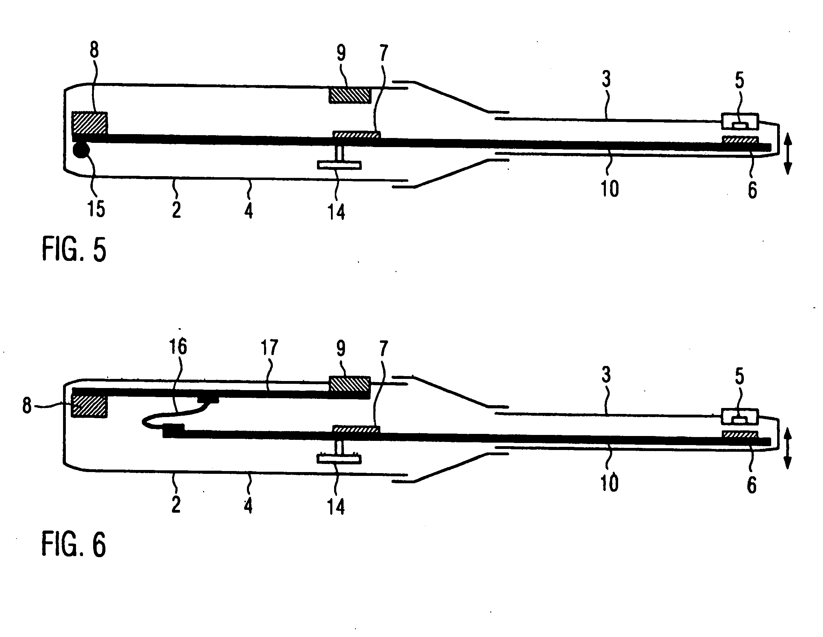

In case of interfacing via a wire, a fixation of the carrier 10 is necessary. The interface also has to be positioned at a certain destination. This can be achieved in one embodiment by fixing the carrier 10 at the housing 2, as can be seen in Fig. 5. Whereas on the one end of the carrier 10 the image sensor 6 is mounted, on the other end the bearing 15 is included. Around this bearing system the carrier 10 can pivot. By pivoting the image sensor 6, it moves away from the lens 5 or moves to the lens 5. The movement can be adjusted by the focus regulation unit 14. In this case, the positioning does not occur perfectly parallel and therefore an angular distortion of the camera results. However, if the carrier is long enough, the distortion is insignificant to reduce image quality significantly.

Another preferable solution comprises two independent circuits which are connected via a cable 16 in order to prevent the interface unit 8 or the user interface such as a button 9, e.g. a snapshot button, from being moved with the carrier 10, on which the camera is mounted. The button 9 therefore is mounted on the support member 17. Another preferable solution consists in an optimized skew, which allows for an improved positioning of the carrier and the image sensor 6, such as a circuit. In this case, the skew has to be optimized in order to allow movement of e.g. 180 degrees, 270 degrees or 360 degrees, a positioning of the necessary distance in order to achieve focus between e.g. 1 cm and infinite, which might equal to a real movement of 3 mm of the circuit, dependent on the selected lens 5.

A further preferable solution in order to improve user comfort comprises instead of using an external available wheel, a slider which controls the positioning of the circuit. Said slider can control, for example, the mentioned wheel directly. The focus regulation unit 14 is therefore adjusted. The use of a slider also allows for the attachment of images in order to demonstrate preferred focused positions such as macro, inter oral and extra oral.

In further improved solutions, such preconfigured solutions are realized in the mechanical systems such as by a nose which blocks if the said position is reached. In a further improved solution, mechanical guides are provided which secure that said movement is performed in a manner that the circuit on which the camera is mounted is moved in a parallel manner.

In a further improved solution, the position is performed by a motor and the user can control the positioning indirectly from buttons.

In a further improved solution, the control of the motor can be performed by a microprocessor such as a microcontroller or a FPGA.

In said solution, preconfigured positioning information can be stored in a memory and the user can control by means of, for example, buttons, in order to reach the next step. This allows for comfortable manual focus.

In an alternative solution, the microprocessor elaborates the positioning information from the object distance which can be retrieved by means of measures such as direct reflection measurement.

An alternative solution comprises an image processing algorithm which provides information on the current image quality. The microprocessor then moves the circuit in the position where image quality is best.

Preferably, the user can change the type of focus mechanism between manual and said autofocus mechanism.

Anyhow, all types of mechanical or electromechanical systems are included which allow for positioning of the circuit on which the camera is mounted.

In one embodiment, a personal computer constitutes the base station. In said case, interfacing can be done, for example, via the USB interface. In an alternative embodiment, interfacing is done via wireless interfacing, by means of, for example, WiFi or Bluetooth, but not limited to the standards.

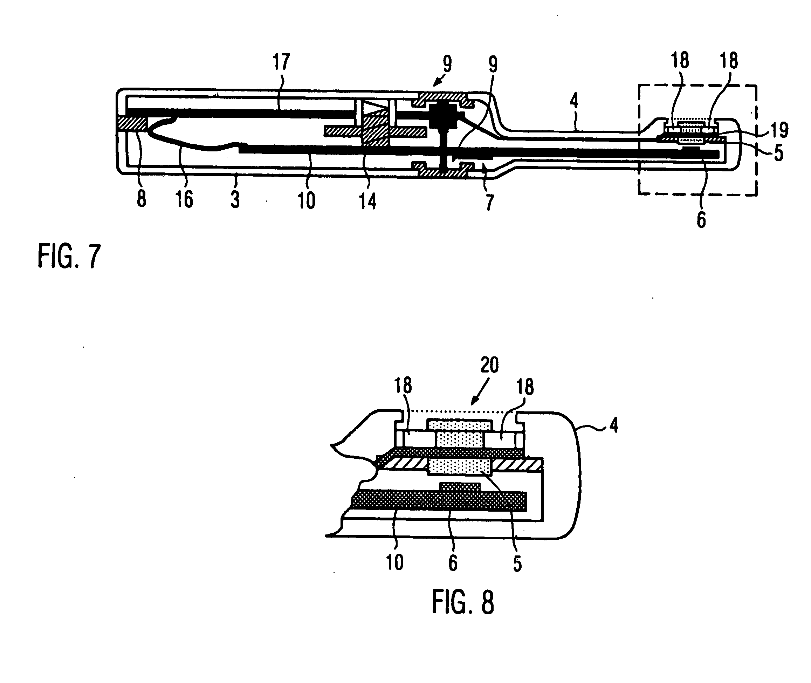

In Fig. 7, a manual focus embodiment of an endoscope 1 according to the invention with such as one carrier 10, one support member 17 and a holder are shown. However, more or even less holding devices might be provided in order to achieve the same results. Further on, the mentioned components might be distributed differently on the separate holding devices and inter connection between the holding devices might be different.

Said first holding device, namely the support member 17, provides user interfaces such as buttons with, for example, snap shot function and interfacing to the base station, such as a USB connector or a wireless communication module. On said holding device, power supply circuitry is also mountable. In a preferable embodiment, snap shot buttons are provided. The snap shot buttons have the reference number 9. One snap shot button is on the top side and one is on the bottom side of the housing 2 of the hand part 4. This is in order to allow simple usage.

On the second holding device, namely the carrier 10, the support member 17 which is connected via the cable 16 to the carrier 10, being the first holding device, whereby to the carrier 10 the image sensor 6 is mounted, and said image processor 7 and associated components. The support member 17 is connected towards the mechanical system that performs the vertical positioning of this holding device. In the preferred embodiment, said mechanical system is an external accessible wheel that turns a screw, which is connected towards the support member 17. Due to the lateral fixation of the support member 17, the rotation maybe transferred into a vertical movement of the carrier 10.

On the third holding device, namely the holder, a number of illumination elements are mountable, such as white top LEDs, also described as lamps 18. The white top LEDs provide illumination especially in case of inter or oral usage. Said third holding device 19 is connected to the support member 17, in order to receive power supply for said illumination elements. In order to allow for a regulation of the luminosity said illumination elements, on the support member 17, is also provided a regulator, such as a potentiometer, to change the power provided to said illumination elements. Said third holding device 19 is mounted in the vicinity of the lens 5. Both, the lens 5 and the illumination elements, lamps 18, are fixed towards the package of the dental camera and the head part 4.

For hygienic reasons, in a preferable solution on top of said illumination elements and said lamps 5, is mounted a transparent element, such as a plexiglass, also known as acrylic glass 20 or a glass disk in order to allow simpler cleaning.

Fig. 8 shows the tip of the head part 3 in more detail.

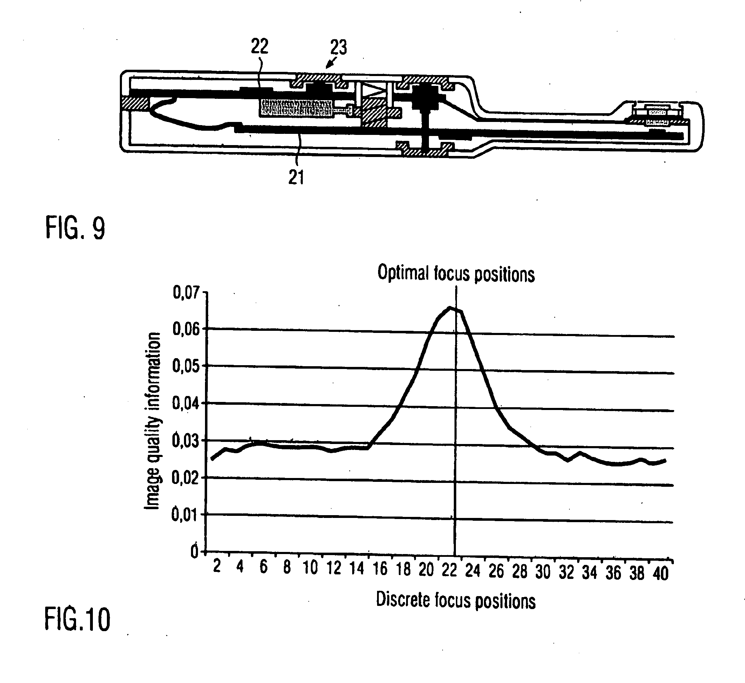

Fig. 9 shows another embodiment of the endoscope 1 according to the invention, with an outer focus modification, having a motor 21, such as a stepper motor, a motor control circuitry 22 and a preferable user interface, such as buttons. Those buttons are part of a mode switch 23.

By means of this mode switch 23 modes can be selected such as manual focus or auto focus. In the manual focus mode the user can control from the user interfaces the desired focus position. The motor control circuitry 22, which might constitute in the simplest case, a power supply circuitry and a micro controller activating the motor, which turns said wheel as described previously in order to control the position of the holding device, namely the carrier 10 and the thereon fixed image sensor 6. In one embodiment a stepper motor is used which allows precise control of a motor 21. A sensor is used which provides information on the exact position of the carrier 10.

The sensor returns information of said wheel which controls the focus, from which the motor control can calculate the actual position of the image sensor 6. In the auto focus mode, said motor control circuitry moves said carrier independently from further user interfaces into the optimal position. Said optimal position might be determined from the distance of the object of the lens 5. This distance can be determined, for example, by means of state of the art reflection methods. However, the head part 4 of the dental camera is a small passive solution, which determines from the actual image quality the position in which the said carrier 10 has to be moved in order to achieve that image quality. Said optimal image quality is, for example, reached by simple Contrast Detection Method and continuing to move a holding device till the best contrast is reached.

The optimal focus position relative to the image quality information, shown as discreet focus positions, is shown in Fig. 9.

Of course, it should be understood that a wide range of changes and modifications can be made to the embodiments described above without departing from the scope of the present invention. It has to be, therefore, understood that the scope of the present invention is not limited to the embodiments disclosed but is rather defined by the appended claims.

## Claims

1. An endoscope (1) with a digital view system, such as a digital camera, with a housing (2) having a longitudinal axis (13), with a lens (5) attached to the housing (2) and with an electronic image sensor (6) positioned in the interior of the housing (2) for converting a picture delivered through the lens (5) Into a digital format, **characterized in that** the image sensor (6) is moveable transverse to the longitudinal axis (13) and the image sensor (6) is fixed to a carrier (10), whereby the carrier (10) is pivotally attached to the housing (2).

2. The endoscope (1), according to claim 1, whereby the image sensor (6) is movable perpendicular to the longitudinal axis (13).

3. The endoscope (1), according to claim 1 or 2, whereby the carrier (10) is parallel to the longitudinal axis (13).

4. The endoscope (1), according to claims 1 - 3, whereby the image sensor (6) comprises a circuit.

5. The endoscope (1), according to claim 1 - 4, whereby a focus regulation unit (14) is comprised in the housing (2)/to change the distance between the lens (5) and the image sensor (6).

6. The endoscope (1), according to claim 5, whereby the focus regulation unit (14) allows a skew of the carrier (10) relative to the lens (5).

7. The endoscope (1), according to at least one of the claims 5 or 6, whereby the focus regulation unit (14) comprises an adjustment element, such as a screw or a wheel, for moving the carrier (10) relative to the housing (2).

8. The endoscope (1), according to at least one of the claims 4 - 7, whereby the endoscope (1) comprises an interface (8) for relaying the data of the circuit to a processing unit (7).

9. The endoscope (1), according to claim 8, whereby the interface (8) has a USB connector and/or a wireless communication module.

10. The endoscope (1), according to at least one of the claims 1 - 9, whereby the housing (2) comprises a head part (3) and a hand part (4), whereby the hand and head parts (3, 4) are preferably positioned consecutively relative to the longitudinal axis (13).

11. The endoscope (1), according to claim 10, whereby the head part (3) has a smaller diameter than the hand part (4), whereby the head part (3) is suitable to be inserted in orifices of a human or animal body.

12. The endoscope (1), according to claims 10 or 11, whereby the lens (5) is fixed to the head part (3) to allow pictures to be transmitted in the interior of the housing (2).

13. The endoscope (1), according to at least one of the claims 10 - 12 whereby, with claim 10 being dependent on claim 6 the focus regulation unit (14) is positioned in the hand part (4).

14. The endoscope (1), according to at least one of the claims 10 - 13 whereby, with claim 10 being dependent on claim 7 the wheel or the screw of the focus regulation unit (14) is positioned on an exterior of the hand part (4) and is in contact with the carrier (10)/ for traversing the carrier (10) relative to the housing (2) of the hand part (4).

15. The endoscope (1), according to at least one of the claims 1 - 14, whereby a support member (17) is positioned in the housing (2)/ separate to the carrier (10).

16. The endoscope (1), according to one of the claims 10 to 15, whereby the carrier (10) comprises an end disposed on the other side of the lens (5) with respect to said image sensor, wherein said end is pivotally attached to an interior section of the hand part (4).

17. The endoscope (1), according to claim 15, whereby the carrier is movable parallel to the support member (17).

18. The endoscope (1), according to claim 8, whereby the interface (8) is attached to a support member (17) and/or an interlinkage is linking the image sensor (6) to the interface (8).

19. The endoscope (1), according to at least one of the claims 1 - 18, whereby at least one lamp (18), preferably a LED, is attached together with the lens (5) in one opening of the housing (2) and preferably covered by a flush covering of the housing (2).

20. The endoscope (1), according to claim 19, whereby the covering is of acrylic glass (20).

21. The endoscope (1), according to at least one of the claims 1 - 20, whereby the endoscope comprises a mode switch (23) to choose between an auto focus and a manual focus mode.

## Patentansprüche

1. Endoskop (1) mit einem digitalen Betrachtungssystem, wie beispielsweise einer Digitalkamera, mit einem Gehäuse (2), das eine Längsachse (13) hat, mit einem an dem Gehäuse (2) angebrachten Objektiv (5) und mit einem elektronischen Bildsensor (6), der im Inneren des Gehäuses (2) positioniert ist, um ein über das Objektiv (5) zugeführtes Bild in ein digitales Format umzuwandeln, **dadurch gekennzeichnet, dass** der Bildsensor (6) quer zu der Längsachse (13) bewegt werden kann und der Bildsensor (6) an einem Träger (10) befestigt ist, wobei der Träger (10) schwenkbar an dem Gehäuse (2) angebracht ist.

2. Endoskop (1) nach Anspruch 1, wobei der Bildsensor (6) senkrecht zu der Längsachse (13) bewegt werden kann.

3. Endoskop (1) nach Anspruch 1 oder 2, wobei der Träger (10) parallel zu der Längsachse (13) ist.

4. Endoskop (1) nach den Ansprüchen 1-3, wobei der Bildsensor (6) eine Schaltung umfasst.

5. Endoskop (1) nach den Ansprüchen 1-4, wobei eine Fokus-Reguliereinheit (14) in dem Gehäuse (2) enthalten ist, um den Abstand zwischen dem Objektiv (5) und dem Bildsensor (6) zu verändern.

6. Endoskop (1) nach Anspruch 5, wobei die Fokus-Reguliereinheit (14) eine Schrägstellung des Trägers (10) relativ zu dem Objektiv (5) ermöglicht.

7. Endoskop (1) nach wenigstens einem der Ansprüche 5 oder 6, wobei die Fokus-Reguliereinheit (14) ein Einstellelement, wie beispielsweise eine Schraube oder ein Rad, zum Bewegen des Trägers (10) relativ zu dem Gehäuse (2) umfasst.

8. Endoskop (1) nach wenigstens einem der Ansprüche 4-7, wobei das Endoskop (1) eine Schnittstelle (8) zum Übertragen der Daten der Schaltung zu einer Verarbeitungseinheit (7) umfasst.

9. Endoskop (1) nach Anspruch 8, wobei die Schnittstelle (8) einen USB-Verbinder und/oder ein Drahtlos-Kommunikationsmodul aufweist.

10. Endoskop (1) nach wenigstens einem der Ansprüche 1-9, wobei das Gehäuse (2) einen Kopfteil (3) und einen Handteil (4) umfasst und der Hand- sowie der Kopfteil (3, 4) vorzugsweise aufeinanderfolgend relativ zu der Längsachse (13) positioniert sind.

11. Endoskop (1) nach Anspruch 10, wobei der Kopfteil (3) einen kleineren Durchmesser hat als der Handteil (4) und der Kopfteil (3) zum Einführen in Öffnungen eines menschlichen oder tierischen Körpers geeignet ist.

12. Endoskop (1) nach den Ansprüchen 10 oder 11, wobei das Objektiv (5) an dem Kopfteil (3) befestigt ist, so dass Bilder in das Innere des Gehäuses (2) übertragen werden können.

13. Endoskop (1) nach wenigstens einem der Ansprüche 10-12, wobei Anspruch 10 von Anspruch 6 abhängig ist, und wobei die Fokus-Reguliereinheit (14) in dem Handteil (4) positioniert ist.

14. Endoskop (1) nach wenigstens einem der Ansprüche 10-13, wobei Anspruch 10 von Anspruch 7 abhängig ist, und wobei das Rad oder die Schraube der Fokus-Reguliereinheit (14) an einer Außenseite des Handteils (4) positioniert ist und in Kontakt mit dem Träger (10) ist, um den Träger (10) relativ zu dem Gehäuse (2) des Handteils (4) zu verstellen.

15. Endoskop (1) nach wenigstens einem der Ansprüche 1-14, wobei ein Trageelement (17) in dem Gehäuse (2) separat von dem Träger (10) positioniert ist.

16. Endoskop (1) nach einem der Ansprüche 10 bis 15, wobei der Träger (10) ein Ende umfasst, das an der anderen Seite des Objektivs (5) in Bezug auf den Bildsensor angeordnet ist, und das Ende schwenkbar an einem Innenabschnitt des Handteils (4) angebracht ist.

17. Endoskop (1) nach Anspruch 15, wobei der Träger parallel zu dem Trägerelement (17) bewegt werden kann.

18. Endoskop (1) nach Anspruch 8, wobei die Schnittstelle (8) an einem Trageelement (17) angebracht ist und/oder eine Zwischenverbindung den Bildsensor (6) mit der Schnittstelle (8) verbindet.

19. Endoskop (1) nach wenigstens einem der Ansprüche 1-18, wobei wenigstens eine Leuchte (18), vorzugsweise eine LED, zusammen mit dem Objektiv (5) in einer Öffnung des Gehäuses (2) angebracht und vorzugsweise mit einer bündigen Abdeckung des Gehäuses (2) abgedeckt ist.

20. Endoskop (1) nach Anspruch 19, wobei die Abdeckung aus Acrylglas (20) besteht.

21. Endoskop (1) nach wenigstens einem der Ansprüche 1-20, wobei das Endoskop einen Modus-Schalter (23) zum Wählen zwischen einem Modus mit Autofokus und einem Modus mit manuellem Fokus umfasst.

## Revendications

1. Endoscope (1) avec un système numérique de vision, tel qu'une caméra numérique, comportant un corps (2) ayant un axe longitudinal (13), une lentille (5) étant fixée au corps (2) et un capteur électronique d'image (6) étant positionné à l'intérieur du corps (2) pour convertir sous un format numérique une image transmise par l'intermédiaire de la lentille (5), **caractérisé en ce que** le capteur d'image (6) est apte à se déplacer transversalement à l'axe longitudinal (13) et est fixé à un support (10), le support (10) étant fixé de façon pivotante au corps (2).

2. Endoscope (1) selon la revendication 1, dans lequel le capteur d'image (6) est apte à se déplacer perpendiculairement à l'axe longitudinal (13).

3. Endoscope (1) selon la revendication 1 ou la revendication 2, dans lequel le support (10) est parallèle à l'axe longitudinal (13).

4. Endoscope (1) selon les revendications 1 à 3, dans lequel le capteur d'image (6) comprend un circuit.

5. Endoscope (1) selon les revendications 1 à 4, dans lequel une unité de réglage de mise au point (14) est contenue dans le corps (2) pour modifier la distance entre la lentille (5) et le capteur d'image (6).

6. Endoscope (1) selon la revendication 5, dans lequel l'unité de réglage de mise au point (14) autorise une inclinaison du support (10) par rapport à la lentille (5).

7. Endoscope (1) selon au moins l'une des revendications 5 et 6, dans lequel l'unité de réglage de mise au point (14) comprend un élément d'ajustement, tel qu'une vis ou une roue, pour déplacer le support (10) par rapport au corps (2).

8. Endoscope (1) selon au moins l'une des revendications 4 à 7, dans lequel l'endoscope (1) comprend une interface (8) servant à relayer les données du circuit vers une unité de traitement (7).

9. Endoscope (1) selon la revendication 8, dans lequel l'interface (8) comporte un connecteur USB et/ou un module de communication sans fil.

10. Endoscope (1) selon au moins l'une des revendications 1 à 9, dans lequel le corps (2) comprend une partie avant (3) et une partie de saisie manuelle (4), les parties avant et de saisie manuelle (3, 4) étant de préférence positionnées de façon consécutive par rapport à l'axe longitudinal (13).

11. Endoscope (1) selon la revendication 10, dans lequel la partie avant (3) présente un diamètre inférieur à celui de la partie de saisie manuelle (4), de sorte que la partie avant (3) est adaptée pour être insérée dans les orifices d'un corps humain ou animal.

12. Endoscope (1) selon la revendication 10 ou 11, dans lequel la lentille (5) est fixée à la partie avant (3) pour permettre aux images d'être transmises dans l'intérieur du corps (2).

13. Endoscope (1) selon au moins l'une des revendications 10 à 12, avec la revendication 10 dépendant de la revendication 6, dans lequel l'unité de réglage de mise au point (14) est positionnée dans la partie de saisie manuelle (4).

14. Endoscope (1) selon au moins l'une des revendications 10 à 13, avec la revendication 10 dépendant de la revendication 7, dans lequel la roue ou la vis de l'unité de réglage de mise au point (14) est positionnée sur une partie extérieure de la partie de saisie manuelle (4) et est en contact avec le support (10) pour déplacer transversalement le support (10) par rapport au corps (2) de la partie de saisie manuelle (4).

15. Endoscope (1) selon au moins l'une des revendications 1 à 14, dans lequel un élément de support (17) est positionné dans le corps (2), séparément du support (10).

16. Endoscope (1) selon l'une des revendications 10 à 15, dans lequel le support (10) comprend une extrémité disposée de l'autre côté de la lentille (5) par rapport audit capteur d'image, ladite extrémité étant fixée de façon pivotante une partie intérieure de la partie de saisie manuelle (4).

17. Endoscope (1) selon la revendication 15, dans lequel le support est apte à se déplacer parallèlement à l'élément de support (17).

18. Endoscope (1) selon la revendication 8, dans lequel l'interface (8) est fixée à un élément de support (17) et/ou un élément l'interliaison relie le capteur d'image (6) à l'interface (8).

19. Endoscope (1) selon au moins l'une des revendications 1 à 18, dans lequel au moins une lampe (18), de préférence une diode LED, est fixée conjointement avec la lentille (5) dans une ouverture du corps (2) et est de préférence recouverte par un couvercle encastré du corps (2).

20. Endoscope (1) selon la revendication 19, dans lequel le couvercle est en verre acrylique (20).

21. Endoscope (1) selon au moins l'une des revendications 1 à 20, dans lequel l'endoscope comprend un commutateur de mode (23) servant à choisir entre un mode de mise au point automatique et un mode de mise au point manuelle.
